# EUROPÄISCHE PATENTSCHRIFT

(11) **EP 0 984 917 B1**
(45) Veröffentlichungstag und Bekanntmachung des Hinweises auf die Patenterteilung: **29.10.2003**
(21) Anmeldenummer: 98925609.4
(22) Anmeldetag: 16.05.1998
(51) Int. Cl.: C07C 67/00

(54) **VERFAHREN ZUR HERSTELLUNG VON ESTERWEICHMACHERN**
METHOD FOR PRODUCING ESTER PLASTICIZERS
PROCEDE DE PREPARATION DE PLASTIFIANTS ESTERIQUES

(30) Priorität: 22.05.1997 DE 19721347
(43) Veröffentlichungstag der Anmeldung: 15.03.2000
(73) Patentinhaber: Celanese Chemicals Europe GmbH, 60439 Frankfurt am Main (DE)
(72) Erfinder: GICK, Wilhelm, D-47199 Duisburg (DE)
(86) Internationale Anmeldenummer: EP9802899
(87) Internationale Veröffentlichungsnummer: WO98052901

(56) Entgegenhaltungen:
- DE-A- 1 945 359
- GB-A- 1 426 057

## Beschreibung

Die Erfindung betrifft ein Verfahren zur Herstellung von Esterweichmachern aus Di- oder Polycarbonsäuren oder deren Anhydride und Alkoholen durch Umsetzung der Ausgangsverbindungen in Gegenwart von Metallkatalysatoren.

Weichmacher finden in großem Umfang und in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihr Löse- und Quellvermögen in physikalische Wechselwirkung. Hierdurch bildet sich ein homogenes System aus, dessen thermoplastischer Bereich gegenüber dem ursprünglichen Polymeren zu niederen Temperaturen verschoben ist mit dem Ergebnis, daß z.B. Formveränderungsvermögen und Elastizität erhöht werden und die Härte verringert wird.

Um Weichmachern möglichst weite Anwendungsgebiete zu eröffnen, müssen sie einer Anzahl Anforderungen genügen. Im Idealfall sollten sie geruchlos, fablos, licht-, kälteund wärmebeständig sein. Überdies erwartet man, daß sie beständig gegen Wasser, schwer brennbar und wenig flüchtig sind und die Gesundheit nicht schädigen. Weiterhin soll die Herstellung der Weichmacher einfach sein und, um ökologischen Ansprüchen zu genügen, unter Vermeidung von Abfallstoffen, wie nicht weiter verwertbaren Nebenprodukten und Schadstoffe enthaltenden Abwässern, erfolgen.

Zu den wichtigsten Weichmachern gehören die Ester von Diund Polycarbonsäuren mit Weichmacheralkoholen, d.h. unverzweigten oder verzweigten primären Alkoholen mit etwa 6 bis 13 Kohlenstoffatomen, die als individuelle Verbindungen oder auch als Gemisch eingesetzt werden. Die Herstellung der Ester erfolgte nach dem klassischen Verfahren durch Umsetzung der Säuren oder Säureanhydride mit einem Alkohol oder einem Gemisch unterschiedlicher Alkohole in Gegenwart einer Säure, vorzugsweise Schwefelsäure als Katalysator. Üblicherweise wird die Alkoholkomponente im Überschuß eingesetzt. Beeinträchtigungen von Farbe und Geruch des Reaktionsproduktes suchte man durch gezielte Auswahl der als Katalysator verwendeten Säure, durch milde Reaktionsbedingungen und durch aufwendige Reinigungsmaßnahmen zu begegnen.

Eine Weiterentwicklung erfuhr die Herstellung von als Weichmacher geeigneten Estern durch den Einsatz metallhaltiger Veresterungskatalysatoren. Geeignet sind z.B. Zinn, Titan, Zirconium, die als feinverteilte Metalle oder zweckmäßig in Form ihrer Salze, Oxide oder löslichen organischen Verbindungen verwendet werden. Diese Katalysatoren sind Hochtemperaturkatalysatoren, sie erreichen ihre volle Aktivität erst bei Veresterungstemperaturen oberhalb 180°C. Beispielhafte Vertreter sind Zinnpulver, Zinn(II)-oxid, Zinn(II)-oxalat, Titansäureester wie Tetraisopropylorthotitanat oder Tetrabutylorthotitanat sowie Zirconiumester wie Tetrabutylzirconat. Besondere Bedeutung haben in technischen Produktionsprozessen Alkyltitanate und Titanchelate, d.h. Titanate von Polyalkoholen, gefunden. Eine derartige Arbeitsweise ist z.B. Gegenstand der US-Patentschrift 5,434,294 und auch in der DE 19 45 359 beschrieben.

Aus EP-A-0 424 767 ist ferner ein Verfahren zur Veresterung von Phthalsäureanhydrid mit Isodecanol in Gegenwart von Tetrabutyltitanat als Katalysator bei 230°C bekannt. Im Anschluß an die Veresterung wird das Reaktionsgemisch mit Sodalösung behandelt und der überschüssige Alkohol abdestilliert. Durch die Behandlung mit der Sodalösung werden die im Reaktionsgemisch vorliegenden Phthalsäurehalbester unter Bildung der entsprechenden Salze neutralisiert. Diese Salze fallen als schleimiger Niederschlag an, der sich nur schlecht und unter hohem zeitlichem und apparativem Aufwand abfiltrieren läßt. Eine Reingewinnung des gewünschten Phthalsäurediesters ist somit mit erhöhten Schwierigkeiten verbunden.

Die modernen Verfahren zur Herstellung von Esterweichmachern genügen somit noch nicht in jeder Beziehung den oben näher beschriebenen Forderungen an den Produktionsprozeß und an das Reaktionsprodukt.

Es bestand daher die Aufgabe, die bekannten Verfahren zu verbessern und durch Abstimmung und Vereinfachung der aufeinanderfolgenden Teilschritte des gesamten Prozesses das Verfahren zu optimieren und die Gewinnung des Reaktionsproduktes in hoher Qualität zu vereinfachen, so daß dieses Reaktionsprodukt möglichst vielseitig angewendet werden kann.

Die Erfindung besteht in einem Verfahren zur Herstellung von Esterweichmachern durch Umsetzung von Di- oder Polycarbonsäuren oder deren Anhydriden mit Alkoholen in Gegenwart eines Titan, Zirconium oder Zinn enthaltenden Katalysators. Es ist dadurch gekennzeichnet, daß man eine Mischung von Säure oder Säureanhydrid und Alkohol zunächst bei 100 bis 160°C unter Entfernung gegebenenfalls gebildeten Wassers miteinander reagieren läßt, die Reaktion unter Zusatz des Katalysators und durch Erhöhen der Temperatur bis auf 250°C zuende führt, das Reaktionsgemisch mit einer wäßrigen Alkali- oder Erdalkalihydroxid-Lösung neutralisiert, darauf den überschüssigen Alkohol abtrennt und den zurückbleibenden Rohester trocknet und filtriert.

Das neue Verfahren zeichnet sich durch große Zuverlässigkeit bei der Realisierung in technischen Anlagen aus. Es ist, auch kontinuierlich, leicht durchzuführen und liefert Weichmacher hoher Reinheit. Besonders bemerkenswert ist die problemlose und vollständige Abtrennung des bei der Umsetzung als Nebenprodukt entstehenden Halbesters, des im Reaktionsgemisch enthaltenen Katalysators und der zur Neutralisation verwendeten Reagenzien. Die restlose Entfernung dieser Nebenprodukte und Hilfsstoffe resultiert u.a. in den ausgezeichneten Farbeigenschaften wie der bemerkenswerten Farbstabilität der Verfahrensprodukte. Hervorzuheben ist weiterhin ihre äußerst geringe Leitfähigkeit, die ihnen breite Anwendung für Kunststoffe auf dem Gebiet der Kabelisolierung eröffnen.

Als Ausgangsstoffe für das erfindungsgemäße Verfahren geeignete Säuren sind aliphatische und aromatische Di- und Polycarbonsäuren. Die aliphatischen Carbonsäuren können gesättigt oder ungesättigt sein und enthalten mindestens vier Kohlenstoffatome. Beispiele für derartige Verbindungen sind Bernsteinsäure, Glutarsäure, Adipinsäure, Korksäure, Azelainsäure, Maleinsäure und Fumarsäure. Unter den aromatischen Carbonsäuren sind insbesondere Phthalsäure und Trimellitsäure zu nennen. Die Säuren können sowohl in reiner Form als auch in Form von Gemischen eingesetzt werden. Statt der Säuren können mit Erfolg auch deren Anhydride Anwendung finden.

Als Alkohole verwendet man insbesondere gerad- oder verzweigtkettige gesättigte aliphatische Verbindungen mit mehr als 6 Kohlenstoffatomen im Molekül. Sie enthalten zumeist eine primäre Hydroxylgruppe, jedoch sind auch sekundäre Alkohole als Reaktionspartner der Säuren nicht ausgeschlossen. Beispiele für Vertreter dieser Verbindungsklasse sind n-Octanol-(1), n-Octanol-(2), 2-Ethylhexanol-(1), n-Nonylalkohol, Decylalkohol und die sogenannten Oxoalkohole, d.h. Gemische gerad- und verzweigtkettiger Alkohole entsprechender Molekülgröße, die durch Oxoreaktion aus Olefinen und anschließende Hydrierung erhalten werden. Auch die Alkohole können als reine Verbindungen oder als Gemische strukturisomerer Verbindungen oder Verbindungen unterschiedlicher Molekülgröße angewandt werden.

Besonders bewährt hat sich die neue Arbeitsweise für die Herstellung von Estern der Phthalsäure mit C₈- bis C₁₂-Alkoholen und unter ihnen insbesondere für die Herstellung von Di(2-ethylhexyl)-phthalat.

Als Veresterungskatalysatoren werden nach dem neuen Verfahren Titan, Zirconium oder Zinn als Metalle in feinverteilter Form oder, bevorzugt, als Verbindungen eingesetzt. Geeignete Verbindungen sind Zinn(II)-oxid, Zinn(II)-oxalat, Phenolate, Acylate und Chelate des Titans und des Zirconiums sowie Ester des Titans und des Zirconiums wie Tetra(isopropyl)orthotitanat, Tetrabutylortholitanat und Tetrabutylzirconat. Die Menge des eingesetzten Katalysators kann sich über einen weiten Bereich erstrecken. Es können sowohl 0,01 Gew.-% als auch mehr als 5 Gew.-% Katalysator, bezogen auf das Reakionsgemisch, verwendet werden. Da größere Katalysatormengen jedoch kaum Vorteile ergeben, beträgt die Katalysatorkonzentration üblicherweise 0,01 bis 1,0, vorzugsweise 0,01 bis 0,5 Gew.-%, jeweils bezogen auf das Reaktionsgemisch.

Obgleich die Veresterung mit stöchiometrischen Mengen Alkohol und Säure vorgenommen werden kann, insbesondere wenn Schleppmittel zur Entfernung des Reaktionswassers verwendet werden, bevorzugt man die Anwendung eines stöchiometrischen Überschusses des Alkohols von 0,05 bis 0,6 mol je mol Di- bzw. Polycarbonsäure oder Säureanhydrid, um eine möglichst vollständige Umsetzung der Säure zu erreichen.

Erfindungsgemäß führt man die Veresterung in zwei Stufen durch. In der ersten Stufe erfolgt, ohne Zusatz eines Katalysators, die Bildung des Halbesters der Dicarbonsäure. Die dabei einzuhaltenden Temperaturen hängen weitgehend von den Einsatzstoffen ab. Befriedigende Reaktionsgeschwindigkeiten werden etwa ab 100°C und vorzugsweise ab 120°C erreicht. Es ist möglich, die Halbesterbildung bei diesen Temperaturen zuendezuführen. Vorteilhafter ist es aber, die Temperatur kontinuierlich bis auf etwa 160°C zu steigern. Bei Verwendung von Carbonsäuren (statt Carbonsäureanhydriden) als Veresterungskomponente wird das entstandene Wasser als Azeotrop mit dem Alkohol aus dem Reaktionssystem ausgetragen, sofern die Reaktionstemperatur oberhalb des Siedepunktes des Azeotrops (d.h. in einem Bereich von 90 bis 100°C unter Normaldruck) liegt. Verlauf und Beendigung der Veresterung kann in diesem Fall über die Bildung des Wassers beobachtet werden. Die Anwendung von vermindertem oder erhöhtem Druck ist nicht ausgeschlossen, wird sich jedoch auf Sonderfälle beschränken. Um das Auftreten von Konzentrationsunterschieden zu unterbinden, empfiehlt es sich, den Reaktorinhalt zu rühren oder zeitweise durchzumischen, z.B. mittels Durchleiten eines Inertgases.

In der zweiten Stufe wird die Veresterung der Di- oder Polycarbonsäuren vollendet. Sie erfolgt in Gegenwart der vorstehend beschriebenen Katalysatoren bei Temperaturen, die oberhalb der in der ersten Stufe eingehaltenen liegen und bis 250°C reichen. Bei dieser Temperatur handelt es sich um einen Richtwert, der zweckmäßig eingehalten wird. Niedrigere Temperaturen können z.B. ausreichen, wenn im speziellen Fall eine genügend hohe Reaktionsgeschwindigkeit erzielt wird oder nur Teilumsätze angestrebt werden. Höhere Temperaturen können eingehalten werden, wenn das Auftreten von Zersetzungsprodukten, die u.a. farbschädigend wirken, auszuschließen ist. Vorteilhaft arbeitet man bei der Siedetemperatur des eingesetzten Alkohols. Die Umsetzung kann in einem eigenen Reaktor oder auch im Reaktionsgefäß der ersten Stufe durchgeführt werden. Während der Reaktion entstandenes Wasser wird als Azeotrop entfernt, wobei der Alkohol die Rolle des Schleppmittels übernimmt. Auch in dieser Reaktionsstufe empfiehlt es sich, den Reaktorinhalt zumindest von Zeit zu Zeit durchzumischen, um Konzentrationsunterschiede auszugleichen und die Entfernung des Reaktionswassers zu beschleunigen.

Nach Beendigung der Reaktion enthält das Reaktionsgemisch neben dem erwünschten Reaktionsprodukt, dem Di- oder Polyester, insbesondere noch teilweise veresterte Dioder Polycarbonsäuren, überschüssigen Alkohol und den Katalysator.

Zur Aufarbeitung des rohen Esterweichmachers wird der Reaktoraustrag zunächst mit Alkali- oder Erdalkalihydroxid neutralisiert. Das alkalische Reagenz gelangt dabei als wäßrige Lösung zur Anwendung, die 5 bis 20, vorzugsweise 10 bis 15 Gew.-% des Hydroxids, bezogen auf die Lösung, enthält. Die Menge des zuzusetzenden Neutralisationsmittels richtet sich nach dem Anteil saurer Komponenten, freie Säure und Halbester im Rohprodukt. Dieser Anteil wird in Form der Säurezahl (nach DIN 53169) bestimmt. Erfindungsgemäß setzt man das alkalische Reagenz in einem Überschuß zu, der der zwei- bis vierfachen Menge entspricht, die stöchiometrisch erforderlich ist, um die vorhandenen H⁺-Ionen zu neutralisieren. Die Verwendung der ausgewählten Hydroxide, unter denen sich Natriumhydroxid besonders bewährt hat, als wäßrige Lösung bestimmter Konzentration und in definiertem Überschuß stellt sicher, daß die aciden Bestandteile des Reaktionsgemischs in kristalliner, ausgezeichnet filtrierbarer Form abgeschieden werden. Gleichzeitig wird der Katalysator weitgehend, zu ebenfalls leicht filtrierbaren Produkten, zersetzt. Die alkalische Behandlung des Rohesters ist an die Einhaltung bestimmter Temperaturen nicht gebunden. Sie erfolgt zweckmäßig unmittelbar im Anschluß an den Veresterungsschritt ohne vorherige Abkühlung des Reaktionsgemisches.

Der Alkali- bzw. Erdalkaliüberschuß, es handelt sich hierbei, bezogen auf das Reaktionsprodukt, nur um geringfügige Mengen, reagiert mit dem Ester zu carbonsaurem Salz, das kristallin anfällt und ohne Schwierigkeiten abfiltriert werden kann.

Im Anschluß an die Neutralisation wird der freie Alkohol aus dem Reaktionsgemisch abgetrennt. Bewährt hat sich für diesen Arbeitsschritt die Wasserdampfdestillation, die in einfacher Form durch Einleiten von Wasserdampf in das Rohprodukt erfolgen kann. Ein Vorteil der Wasserdampfdestillation ist, daß in ihrem Verlauf auch noch letzte Katalysatorreste zerstört und in beguem filtrierbare Hydrolyseprodukte umgewandelt werden. In diesem Zusammenhang kann es vorteilhaft sein, dem Reaktionsgemisch vor der Destillation ein großflächiges Adsorbens, z.B. Aktivkohle, zuzusetzen, um die Abscheidung der Katalysatorfolgeprodukte zu erleichtern.

An die Entfernung des freien Alkohols schließt sich die Trocknung des Esters an. In einer besonders einfachen und wirksamen Ausführungsform dieses Arbeitsschritts leitet man hierzu ein inertes Gas durch das Produkt. Darauf wird der Rohester filtriert, um ihn von den Feststoffen, den Salzen von (gegebenenfalls teilweise veresterten) Carbonsäuren, Hydrolyseprodukten des Katalysators und dem Adsorbens, zu befreien. Die Filtration erfolgt in konventionellen Filtrierapparaten bei normaler Temperatur oder bei Temperaturen bis zu 150°C. Die Filtration kann durch gängige Filterhilfsmittel wie Cellulose, Kieselgel, Kieselgur, Holzmehl, unterstützt werden. Thr Einsatz ist jedoch auf Ausnahmefälle beschränkt.

Das erfindungsgemäße Verfahren senkt die Zeit zur Abtrennung der im Rohester enthaltenen Feststoffe drastisch auf Werte, die seine wirtschaftliche Durchführung im technischen Maßstab sicherstellen und vermeidet die Bildung nach Menge und Zusammensetzung ökologisch bedenklicher Abwässer.

Die nach dem beanspruchten Prozeß erhaltenen Ester weisen eine hervorragende Qualität auf, die auch hohen Ansprüchen in jeder Hinsicht genügt.

## Patentansprüche

1. Verfahren zur Herstellung von Esterweichmachern durch Umsetzung von Di- oder Polycarbonsäuren oder deren Anhydriden mit Alkoholen in Gegenwart eines Titan, Zirconium oder Zinn enthaltenden Katalysators, **dadurch gekennzeichnet, daß** man eine Mischung von Säure oder Säureanhydrid und Alkohol zunächst bei 100 bis 160°C unter Entfernung gegebenfalls gebildeten Wassers miteinander reagieren läßt, die Reaktion unter Zusatz des Katalysators und durch Erhöhen der Temperatur bis auf 250°C zuende führt, das Reaktionsgemisch mit einer wäßrigen Alkali- oder Erdalkalihydroxid-Lösung neutralisiert, darauf den überschüssigen Alkohol abtrennt, den zurückbleibenden Rohester trocknet und filtriert.

2. Verfahren nach Anspruch 1, **dadurch gekennzeichnet, daß** der Katalysator Tetra(isopropyl)orthotitanat, Tetrabutylorthotitanat oder Tetrabutylzirconat ist.

3. Verfahren nach Anspruch 1 oder 2, **dadurch gekennzeichnet, daß** man den Alkohol in einem stöchiometrischen Überschuß von 0,05 bis 0,6 mol je mol Di- bzw. Polycarbonsäure einsetzt.

4. Verfahren nach einem oder mehreren der Ansprüche 1 bis 3, **dadurch gekennzeichnet, daß** während der Umsetzung entstehendes Reaktionswasser als Azeotrop mit dem verwendeten Alkohol aus dem Reaktionsgemisch entfernt wird.

5. Verfahren nach einem oder mehreren der Ansprüche 1 bis 4, **dadurch gekennzeichnet, daß** man die Umsetzung bei der Siedetemperatur des verwendeten Alkohols zuendeführt.

6. Verfahren nach einem oder mehreren der Ansprüche 1 bis 5, **dadurch gekennzeichnet, daß** die zur Neutralisation des Reaktionsgemisches angewandte Alkalioder Erdalkalihydroxid-Lösung 5 bis 20 Gew.-% Hydroxid, bezogen auf die Lösung, enthält.

7. Verfahren nach Anspruch 6, **dadurch gekennzeichnet, daß** die Alkali- oder Erdalkalihydroxid-Lösung 10 bis 15 Gew.-% Hydroxid, bezogen auf die Lösung, enthält.

8. Verfahren nach einem oder mehreren der Ansprüche 1 bis 7, **dadurch gekennzeichnet, daß** die Alkali- bzw. Erdalkalihydroxid-Lösung in einem Überschuß angewandt wird, der der zwei- bis vierfachen Menge entspricht, die stöchiometrisch erforderlich ist, um die im Reaktionsgemisch vorhandenen H⁺-Ionen zu neutralisieren.

9. Verfahren nach einem oder mehreren der Ansprüche 1 bis 8, **dadurch gekennzeichnet, daß** überschüssiger Alkohol aus dem Reaktionsgemisch durch Wasserdampfdestillation gegebenenfalls in Gegenwart eines Adsorbens abgetrennt wird.

10. Verfahren nach einem oder mehreren der Ansprüche 1 bis 9, **dadurch gekennzeichnet, daß** als Ausgangsstoffe Phthalsäure und 2-Ethylhexanol miteinander umgesetzt werden.

## Claims

1. A process for preparing ester plasticizers by reacting dicarboxylic or polycarboxylic acids or their anhydrides with alcohols in the presence of a titanium-, zirconium- or tin-containing catalyst, which comprises allowing a mixture of acid or acid anhydride and alkohol to react first at from 100 to 160°C while removing any water formed, completing the reaction by addition of the catalyst and increasing the temperature to 250°C, neutralizing the reaction mixture with an aqueous alkali metal hydroxide or alkaline earth metal hydroxide solution, then separating off the excess alcohol and drying and filtering the remaining crude ester.

2. The process as claimed in claim 1, wherein the catalyst is tetra(isopropyl) orthotitanate, tetrabutyl orthotitanate or tetrabutyl zirconate.

3. The process as claimed in claim 1 or 2, wherein the alkohol is used in a stoichiometric excess of from 0.05 to 0.6 mole per mole of dicarboxylic or polycarboxylic acid.

4. The process as claimed in one or more of claims 1 to 3, wherein water of reaction formed during the reacting is removed from the reaction mixture as an azeotrope with the alcohol used.

5. The process as claimed in one or more of claims 1 to 4, wherein the reaction is completed at the boiling point of the alcohol used.

6. The process as claimed in one or more of claims 1 to 5, wherein the alkali metal hydroxide or alkaline earth metal hydroxide solution employed for neutralizing the reaction mixture contains from 5 to 20% by weight of hydroxide, based on the solution.

7. The process as claimed in claim 6, wherein the alkali metal hydroxide or alkaline earth metal hydroxide solution contains from 10 to 15 % by weight of hydroxide, based on the solution.

8. The process as claimed in one or more of claims 1 to 7, wherein the alkali metal hydroxide or alkaline earth metal hydroxide solution is employed in an excess which corresponds to from two to four times the amount which is stoichiometrically required to neutralize the H⁺ ions present in the reaction mixture.

9. The process as claimed in one or more of claims 1 to 8, wherein excess alcohol is separated from the reaction mixture by steam distillation, if desired in the presence of an adsorbent.

10. The process as claimed in one or more of claims 1 to 9, wherein phthalic acid and 2-ethylhexanol as starting materials are reacted with one another.

## Revendications

1. Procédé en vue de la fabrication de plastifiants d'esters par réaction d'acides dicarboxyliques ou d'acides polycarboxyliques ou de leurs anhydrides avec des alcools en présence d'un catalyseur contenant du titane, du zirconium ou de l'étain, **caractérisé en ce que** l'on laisse tout d'abord réagir ensemble un mélange d'acide ou d'anhydride d'acide et d'alcool à une température de 100 à 160ºC avec élimination, le cas échéant, de l'eau formée, **en ce que** l'on achève la réaction par addition du catalyseur et par élévation de la température jusqu'à environ 250ºC, **en ce que** l'on neutralise le mélange réactionnel à l'aide d'une solution d'hydroxydes d'alcalins ou d'alcalino-terreux, en ce l'on élimine ensuite l'alcool en excès et **en ce que** l'on sèche et filtre l'ester brut résiduel.

2. Procédé selon la revendication 1, **caractérisé en ce que** le catalyseur est l'orthotitanate de tétra(isopropyle), l'orthotitanate de tétrabutyle ou le zirconate de tétrabutyle.

3. Procédé selon la revendication 1 ou 2, **caractérisé en ce que** l'on utilise l'alcool dans un excès stoechiométrique de 0,05 à 0,6 mole par mole d'acide dicarboxylique ou polycarboxylique.

4. Procédé selon l'une quelconque des revendications 1 à 3, **caractérisé en ce que** l'on élimine du mélange réactionnel, en tant qu'azéotrope avec l'alcool utilisé, l'eau de réaction qui se forme pendant la réaction.

5. Procédé selon l'une ou plusieurs des revendications 1 à 4, **caractérisé en ce que** l'on achève la réaction à la température d'ébullition de l'alcool utilisé.

6. Procédé selon l'une ou plusieurs des revendications 1 à 5, **caractérisé en ce que** la solution d'hydroxyde d'alcalins ou d'alcalino-terreux, utilisé en vue de la neutralisation du mélange réactionnel, contient de 5 à 20% en poids d'hydroxyde, par rapport à la solution.

7. Procédé selon la revendication 6, **caractérisé en ce que** la solution d'hydroxyde d'alcalins ou d'alcalino-terreux contient de 10 à 15% en poids d'hydroxyde, par rapport à la solution.

8. Procédé selon l'une quelconque des revendications 1 à 7, **caractérisé en ce que** la solution d'hydroxyde d'alcalins ou d'alcalino-terreux est utilisée dans un excès qui correspond à la quantité, double à quadruple, de celle qui est nécessaire du point de vue stoechiométrique, pour neutraliser les ion H+ présents dans le mélange réactionnel.

9. Procédé selon l'une ou plusieurs des revendications 1 à 8, **caractérisé en ce que** l'alcool en excès est éliminé du mélange réactionnel par distillation à la vapeur d'eau, le cas échéant en présence d'un agent adsorbant.

10. Procédé selon l'une quelconque des revendications 1 à 9, **caractérisé en ce que** l'on fait réagir ensemble, en tant que matériaux de départ, l'acide phtalique et le 2-éthylhexanol.
